# EUROPÄISCHE PATENTSCHRIFT

(11) **EP 1 329 229 B1**
(45) Veröffentlichungstag und Bekanntmachung des Hinweises auf die Patenterteilung: **16.11.2005**
(21) Anmeldenummer: 03405002.1
(22) Anmeldetag: 03.01.2003
(51) Int. Cl.: A61L 27/12, A61L 27/56

(54) **Poröses Calciumphosphat-Knochenersatzmaterial**
Porous calcium phosphate bone replacement material
Matériau poreux à base de phosphate de calcium pour la substitution osseuse

(30) Priorität: 16.01.2002 DE 10201340
(43) Veröffentlichungstag der Anmeldung: 23.07.2003
(73) Patentinhaber: Biovision Gmbh, 98693 Ilmenau (DE); Fraunhofer-Gesellschaft zur Förderung der angewandten Forschung e.V., 80686 München (DE)
(72) Erfinder: Leuner, Barbara, 98693 Ilmenau (DE); Reif, Dieter, 98553 Altendammbach (DE); Apel, Hans, 98693 Ilmenau (DE); Heymer, Heike, 01217 Dresden (DE); Standke, Gisela, 01099 Dresden (DE); Adler, Joerg, 01662 Meissen (DE)
(74) Vertreter: Frei, Alexandra Sarah

(56) Entgegenhaltungen:
- WO-A-99/16479
- GB-A- 2 078 696
- US-A- 3 090 094
- US-B1- 6 235 225
- SAGGIO-WOYANSKY J ET AL: "PROCESSING OF POROUS CERAMICS" AMERICAN CERAMIC SOCIETY BULLETIN, AMERICAN CERAMIC SOCIETY. COLUMBUS, US, Bd. 71, Nr. 11, 1. November 1992 (1992-11-01), Seiten 1674-1682, XP000349841 ISSN: 0002-7812

## Beschreibung

Die Erfindung betrifft ein Knochen-Ersatzmaterial nach dem Oberbegriff des ersten, unabhängigen Patentanspruchs und ein Verfahren zur Herstellung des Knochen-Ersatzmaterials nach dem Oberbegriff des entsprechenden, unabhängigen Patentanspruchs. Das Knochen-Ersatzmaterial ist eine Schaumkeramik aus Calziumphosphat und weist eine hohe, offene Porosität auf.

Calziumphosphate, insbesondere Calzium-Metaphosphat (Ca(PO₃)₂), Di-Calziumphosphat (Ca₂P₂O₇), Tri-Calziumphosphat (Ca₃(PO₄)₂), Hydroxyapatit (Ca₅(PO₄)₃OH) und Tetra-Calziumphosphat (Ca₄(PO₄)₂O) sind als Knochen-Ersatzmaterialien geeignet, insbesondere wegen ihrer hohen biologischen Verträglichkeit, wegen ihrer osteoinduktiven Wirkung und teilweise (Tri-Calziumphosphat) auch wegen ihrer biologischen Abbaubarkeit.

Versuche haben gezeigt, dass die genannten Calziumphosphate als Knochen-Ersatzmaterialien insbesondere dann geeignet sind, wenn sie eine offen poröse Struktur aufweisen mit Poren, deren Abmessungen 100µm nicht unterschreiten und deren Volumen einen Anteil des Gesamtvolumens von mindestens 70% ausmacht. Eine derartige Porosität begünstigt das Einwachsen von nativem Knochengewebe in das Knochen-Ersatzmaterial, was zu einer guten Verbindung zwischen nicht resorbierbarem Ersatzmaterial und Gewebe oder zu einem schnellen und regelmässigen Abbau von resorbierbarem Ersatzmaterial führt.

Es ist bekannt, dass Schaumkeramiken aus Calziumphosphat, die derartige Porositäten aufweisen und sich deshalb als Knochen-Ersatzmaterialien eignen, nach dem sogenannten Schwartzwalder-Verfahren (US-3090094) herstellbar sind, welches Verfahren ursprünglich zur Herstellung von hitzebeständigen, keramischen Filtermaterialien und Katalysatorträgern entwickelt wurde. Nach diesem Verfahren wird ein retikulierter Kunststoffschaum mit einem Keramik-Schlicker derart getränkt, dass die Schaumstrukturen möglichst vollständig mit Schlicker beschichtet sind, dass aber die Poren der Schaumstruktur nicht mit Schlicker gefüllt sind. Dann wird der beschlikkerte Schaum getrocknet und schliesslich einer thermischen Behandlung ausgesetzt, in der zuerst der Kunststoffschaum ausgebrannt und dann das verbleibende Keramikmaterial gesintert wird. Es entsteht dabei eine offenporige Schaumkeramik, die ein Abbild des Kunststoffschaumes darstellt und im wesentlichen aus einem Gerüst von hohlen Stegen mit etwa dreieckigen Querschnitten besteht, wobei die Stege ein Gebilde von im wesentlichen Pentagondodekaeder-förmigen, ineinander übergehenden Hohlräumen bilden.

Die Porosität einer nach dem Schwartzwalder-Verfahren hergestellten Schaumkeramik ist in weiten Grenzen einstellbar insbesondere durch die Verwendung eines entsprechend porösen Kunststoffschaumes und durch die Menge an Keramikmaterial, die mit dem Schlicker in den Schaum eingebracht wird.

Retikulierte Kunststoffschäume, das heisst Kunststoffschäume aus denen zur Herstellung einer offenen Porosität in einer thermischen oder chemischen Nachbehandlung die dünnen Häutchen zwischen den Schaumzellen beseitigt worden sind, eignen sich für die Herstellung von Knochen-Ersatzmaterialien nach dem Schwartzwalder-Verfahren, wenn sie eine Porosität im Bereiche von etwa 45 bis 90 ppi (45-90 Poren pro Inch oder etwa 18 bis 36 Poren pro cm) aufweisen. Dies entspricht einer Porengrösse (Zellweite) von ca. 300 bis 1100µm. Retikulierte Polyurethanschäume mit der genannten Porosität sind im Handel erhältlich.

Der Schlicker enthält Calziumphosphat als feines Pulver mit einer Partikelgrösse im Mikrometer-Bereich, welches Pulver in Wasser dispergiert wird. Ferner enthält der Schlicker ein Dispergiermittel und ein Bindemittel und gegebenenfalls weitere Zusätze wie beispielsweise einen Entschäumer. Die Zusammensetzung des Schlickers wird derart eingestellt, dass er strukturviskos (thixotrop) ist und dadurch eine stabile und gleichmässige Beschichtung des Kunststoffschaumes erlaubt.

Die genaue Zusammensetzung des Schlickers muss für jede Anwendung experimentell ermittelt werden, wobei die folgenden Gesichtspunkte zu beachten sind:
- je weniger viskos und/oder strukturviskos der Schlicker ist, desto weniger Schlicker kann auf den Kunststoffschaum aufgebracht werden;
- Schlicker mit einer zu hohen Viskosität kann nicht befriedigend in den Kunststoffschaum eingebracht werden;
- je mehr Schlicker im Kunststoffschaum vorhanden ist, desto grösser ist die Wahrscheinlichkeit, dass Poren mit Schlicker ganz gefüllt sind;
- je mehr Keramikmaterial im Kunststoffschaum vorhanden ist, desto dicker werden die Streben in der Schaumkeramik und desto mehr Festigkeit erhält dieser;
- je grösser die Keramikpartikel sind, desto mehr Keramikmaterial kann der Schlicker bei gleicher Viskosität enthalten;
- je grösser die Partikel sind, desto weniger werden sie bei der Sinterung miteinander verbunden und desto kleiner ist die Festigkeit der Schaumkeramik;
- je grösser die Partikel sind, desto weniger schwindet die Schaumkeramik bei der Sinterung.

Je nach Anwendung ist der eine oder der andere Gesichtspunkt wichtig, was sich in der für eine spezifische Anwendung optimalen Zusammensetzung des Schlickers widerspiegelt. Für alle Fälle gilt, dass die Menge an Keramikmaterial, die in den Kunststoffschaum eingebracht werden kann, gegen oben begrenzt ist.

Der Kunststoffschaum wird mit dem Schlicker getränkt vorteilhafterweise durch mehrmaliges Zusammenpressen und Entspannen des im Schlicker eingetauchten Schaumes, so dass der Schaum möglichst regelmässig benetzt wird. Dann wird überschüssiger Schlicker aus dem Kunststoffschaum entfernt durch Zusammenpressen des Schaumes beispielsweise mit Hilfe von Quetschwalzen oder durch Absaugen des Schlickers aus dem Schaum.

Die Trocknung des Schaumes erfolgt bei Temperaturen unterhalb der für die Ausbrennung und Sinterung notwendigen Temperaturen, vorzugsweise unter 100°C. Die Ausbrennung wird bei auf den Kunststoff abgestimmten Temperaturen (Polyurethan: bis ca. 600°C) und die Sinterung bei auf das Keramikmaterial abgestimmten Temperaturen durchgeführt.

Entsprechende Untersuchungen zeigen, dass Keramikschäume, die nach dem Schwartzwalder-Verfahren hergestellt sind, eine Festigkeit aufweisen, die kleiner ist als nach entsprechenden Berechnungen erwartet werden könnte. Es wird vermutet, dass dieses Defizit an Festigkeit mindestens teilweise auf Rissbildung während der thermischen Behandlung zurückzuführen ist, beispielsweise bedingt durch die verschiedenen Wärmeausdehnungen von Kunststoffgerüst und Keramikmaterial, welche Verschiedenheit insbesondere während der ersten Phase der thermischen Behandlung (vor und während dem Ausbrand des Kunststoffschaumes) zu Rissbildung führt. Es scheint, dass diese Rissbildung auch mit sehr sorgfältig durchgeführten thermischen Behandlungen nicht vermieden werden kann.

Dieser Effekt und die an sich kleine Festigkeit von Calziumphosphaten führen dazu, dass die nach dem Schwartzwalder-Verfahren hergestellten Calziumphosphat-Schäume in Bezug auf ihre chemischen und strukturellen Eigenschaften zwar ausgezeichnete Knochen-Ersatzmaterialien liefern, dass diese Materialien aber wegen ihrer geringen Festigkeit nur an unbelasteten Defektstellen einsetzbar sind und für Knochendefekte in belasteten oder teilbelasteten Regionen nicht verwendet werden können. Ferner sind sie nicht ohne Probleme handhabbar und noch schwieriger oder überhaupt nicht mechanisch bearbeitbar. Aus diesem Grunde müssen Formkörper meist als solche, das heisst unter Verwendung entsprechend geformter Kunststoffschaum-Stücke hergestellt werden, was sehr aufwendig ist. Wenn das Material eine grössere Festigkeit hätte, könnte es universaler eingesetzt werden und könnten Formkörper im Operationssaal durch mechanische Bearbeitung aus grösseren Stükken geformt werden, was eine bedeutend bessere und einfachere Anpassung an zu füllende Defektstellen erlauben würde.

Für eine Erhöhung der Festigkeit von nach dem Schwartzwalder-Verfahren hergestellten Schaumkeramiken werden verschiedene Massnahmen vorgeschlagen (aus "Processing of Porous Ceramics", J. Saggio-Woyansky et al., American Ceramic Society Bulletin, vol. 71, Nr. 11, Seiten 1674-1682, 1992):
- Zusatz von anorganischen Fasern zum Schlicker;
- Zusatz von Mitteln, die die Struktur des Kunststoffschaumes schon vor dem Ausbrennen teilweise auflösen (Reduktion der Spannungsrisse);
- Vorbehandlung des Kunststoffschaumes mit einem Adhesiv und organischen Fasern (Erzielung von dickeren Schlicker-Beschichtungen).

Für die Herstellung von Keramikschäumen, die als Knochen-Ersatzmaterialien verwendet werden sollen, sind die oben genannten Methoden zur Erhöhung der Festigkeit nicht vorteilhaft oder nicht möglich, denn es müssen dem Keramikmaterial weitere Substanzen zugefügt werden, die nicht mit der Anwendung kompatibel und/oder nicht abbaubar sein können oder die zu nicht mit der Anwendung kompatiblen Rückständen führen können.

Zur Herstellung von als Knochen-Ersatzmaterial verwendbaren, hochporösen Keramikmaterialien mit erhöhten Festigkeiten schlägt die Publikation WO-99/16479 unter anderem vor, nach dem Schwartzwalder-Verfahren eine Schaumkeramik aus Zirkonoxid oder Aluminiumoxid herzustellen und diese dann in einer zweiten Beschlikkerung mit einem Calziumphosphat-Schlicker zu beschichten und nochmals einer Trocknung und Sinterung zu unterziehen. Das innere Gerüst aus Zirkon- oder Aluminiumoxid übernimmt dabei die Funktion der Festigkeit, die Calziumphosphat-Beschichtung die Funktion der Osteoinduktivität. Der so hergestellte Schaum weist eine bedeutend höhere Festigkeit auf als vergleichbare Schäume, die nur aus Calziumphosphat bestehen. Andererseits macht das Verfahren aber nur Sinn für nichtabbaubere Calziumphosphate. Vollständig abbaubare Knochen-Ersatzmaterialien sind auf diese Weise nicht herstellbar.

Die Erfindung stellt sich nun die Aufgabe, als Knochen-Ersatzmaterial verwendbare, im wesentlichen nach dem Schwartzwalder-Verfahren hergestellte Schaumkeramiken aus Calziumphosphat, insbesondere aus biologisch abbaubarem Tri-Calziumphosphat zu schaffen, die eine für die Anwendung geeignete Porosität haben, deren Festigkeit aber gegenüber bekannten derartigen Schaumkeramiken markant erhöht ist. Ferner ist es die Aufgabe der Erfindung, ein Verfahren zur Herstellung solcher Schaumkeramiken aus Calziumphosphat anzugeben. Die nach dem erfindungsgemässen Verfahren hergestellten Schaumkeramiken sollen sich insbesondere eignen als Implantate für belastete oder teilbelastete Körperregionen in Form von Formstücken und für eine Herstellung derartiger Formstücke durch mechanische Bearbeitung.

Diese Aufgabe wird gelöst durch das Knochen-Ersatzmaterial und das Verfahren zu seiner Herstellung, wie sie in den Patentansprüchen definiert sind.

Das erfindungsgemässe Knochen-Ersatzmaterial besteht aus Calziumphosphat, insbesondere aus dem biologisch abbaubaren Tri-Calziumphosphat. Es enthält im wesentlichen keine anderen Stoffe. Es hat die typische Struktur, die sich aus dem Schwartzwalder-Verfahren ergibt, es hat eine offene Porosität von mindestens 70 Vol.% und eine Festigkeit, die als Bruchlast mit einem Prüfstempel mit einem Durchmesser von 20mm ermittelt wird und die mindestens 550 N beträgt.

Das erfindungsgemässe Knochen-Ersatzmaterial wird erzeugt durch ein Schwartzwalder-Verfahren, das insbesondere im Hinblick auf eine Reduktion der Rissbildung bei der Wärmebehandlung weiterentwickelt wurde. Für diese Weiterentwicklung zeigen sich im wesentlichen zwei Ansätze, die vorteilhafterweise kombiniert angewendet werden.

Einerseits wird die Festigkeit des Schaummaterials erhöht, wenn das Keramikmaterial in einer vollständig kristallinen und phasenreinen Form (mindestens 98%) eingesetzt wird und die Sinterung der Schaumstruktur derart gesteuert wird, dass die Kristallstruktur des eingesetzten Materials erhalten bleibt. Dies wird beispielsweise erreicht durch eine Vorsinterung des zu verwendenden Materials bei einer durch die gewünschte Kristallstruktur vorgegebenen Vorsintertemperatur und eine Sinterung der Schaumstruktur bei einer Temperatur, die niedriger ist als die Vorsintertemperatur und bei der die Kristallstruktur stabil ist. Durch diese Massnahme ergibt sich bei der Sinterung des Keramikmaterials ein Minimum an Strukturumwandlungen und ein Minimum von durch Strukturumwandlungen bedingter Rissbildung.

Andererseits wird die Festigkeit des Schaummaterials erhöht, wenn das Schwartzwalder-Verfahren unter Verwendung desselben Keramikmaterials im wesentlichen zwei mal nacheinander durchgeführt wird, wobei beim zweiten Mal nicht mehr der Kunststoffschaum, der vom Keramikmaterial verschiedene thermische Eigenschaften hat, als Gerüst dient sondern bereits ein gleiches Keramikmaterial. Bei der zweiten Sinterung treten dadurch weniger Spannungen auf als bei der ersten und zudem kann der zweite Materialauftrag eine Reparatur von Spannungsrissen aus der ersten Sinterung bewirken.

Es zeigt sich, dass ein Einsatz von vollständig kristallinem und phasenreinem Keramikmaterial bei einem einzigen Verfahrensdurchgang ähnliche Festigkeiten ergibt wie ein Einsatz von nur mehrheitlich kristallinem Keramikmaterial bei einem zweimaligen Verfahrensdurchgang. Der Einsatz von mindestens mehrheitlich kristallinem Keramikmaterial führt zu einer feinkristallinen, zerklüfteten Sinterstruktur, die das Anhaften von Zellen und nativem Gewebe begünstigt, was einen weiteren Vorteil des erfindungsgemässen Knochen-Ersatzmaterials darstellt. Der Einsatz von mehrheitlich amorphem Material empfiehlt sich nicht, denn er führt zu einer bedeutend gröberen Sinterstruktur und zu einer völlig ungenügenden Festigkeit. Ferner ist bei der Verwendung von mehrheitlich amorphem Material eine sehr viel grössere Schwindung bei der Sinterung zu beobachten als dies bei Verwendung von mindestens mehrheitlich kristallinem Material der Fall ist.

Das Verfahren zur Herstellung des erfindungsgemässen Knochen-Ersatzmaterials weist also alle Schritte des Schwartzwalder-Verfahrens auf. Dabei wird ein mehrheitlich (>70%) bis vollständig (>98%) kristallines und phasenreines Keramikmaterial eingesetzt und die Sintertemperatur auf eine Beibehaltung der Kristallstruktur des Ausgangsmaterials ausgerichtet und wird insbesondere bei Verwendung von nur mehrheitlich kristallinem Material der nach dem Schwartzwalder-Verfahren hergestellte Calziumphosphat-Schaum zusätzlich ein zweites Mal mit im wesentlichen demselben Material beschlickert, getrocknet und gesintert.

Die geforderte Festigkeit wird erreicht durch Verwendung von mehrheitlich bis vollständig kristallinem Keramikmaterial und zweimaliger Durchführung des Schwartzwalder-Verfahrens oder durch Verwendung von vollständig kristallinem und phasenreinem Keramikmaterial und einmaliger Durchführung des Schwartzwalder-Verfahrens. Dabei ist es weiter vorteilhaft, insbesondere bei der Variante mit nur einem Durchgang, als Ausgangsprodukt ein Gemisch von Keramikpartikeln zweier Grössenordnungen zu verwenden, beispielsweise 20-40 Gewichtsteile von Partikeln mit d₅₀ = 1-5µm und 80-60 Gewichtsteile von Partikeln mit d₅₀ = 7-15µm.

Bei zweifacher Durchführung des Schwartzwalder-Verfahrens wird für die zweite Beschlickerung ein Schlicker verwendet, der wiederum ein mindestens teilweise kristallines (vorgesintertes) Calziumphosphat enthält, vorteilhafterweise dasselbe Calziumphosphat wie der erste Schlicker. Dabei enthält der zweite Schlicker weniger Keramikmaterial als der erste Schlicker. Gegebenenfalls kann die Zweitbeschlickerung auch wiederholt werden, das heisst kann eine Drittbeschlickerung mit einer dritten Trocknung und Sinterung durchgeführt werden.

Da der in der Zweitbeschlickerung zu bearbeitende Schaum nicht wie der ursprünglich eingesetzte Kunststoffschaum elastisch verformbar ist, kann der Schlicker nach der zweiten Beschlickerung nicht mehr durch Zusammenpressen und Entlasten eingebracht und kann überflüssiger Schlicker nicht mehr durch Auspressen entfernt werden. Der Schlicker wird statt dessen aktiv durch den Schaum gesaugt.

Es zeigt sich, dass ein zweimal beschlickerter Calziumphosphat-Schaum um ca. 30 bis 50% mehr Festigkeit aufweist als derselbe Calziumphosphatschaum vor der Zweitbeschlickerung, während der Volumenanteil der Poren um 5 bis 15% reduziert ist. Die Festigkeitszunahme ist bedingt durch die Vergrösserung der Strebenquerschnitte im Schaum und vermutlich auch durch ein Ausfüllen oder Verschliessen von Rissen in den bei der ersten Beschlickerung erstellten Streben. Dadurch dass die in der ersten und in der zweiten Beschlickerung entstehenden Schichten im wesentlichen aus denselben Materialien bestehen, sind die Spannungen, die bei der zweiten thermischen Behandlung entstehen, bedeutend kleiner als bei der ersten thermischen Behandlung, so dass auch weniger oder keine neuen Spannungsrisse in den Streben entstehen. Die zweimalige Beschlickerung macht es weniger wichtig, bei der ersten Beschlickerung so viel Keramikmaterial wie möglich aufzubringen, was Raum für andere Kriterien bei der Schlickerherstellung ergibt und durch kleinere Schichtdicken die Rissbildung schon bei der ersten Beschlickerung reduzieren kann.

Die Festigkeit von nach dem Schwartzwalder-Verfahren hergestellten Schaumkeramiken lässt sich nicht wie für kompakte Materialien durch eine Drei- oder Vierpunktbiegeprüfung eines definierten Probekörpers ermitteln. Die Festigkeit von Schaumkeramiken kann mit Hilfe entsprechender mathematischer Modelle und räumlichen Abmessungen der Schaumstruktur aus der Festigkeit des entsprechenden kompakten Materials abgeleitet werden. Solche Methoden sind aufwendig und ungeeignet. Aus diesem Grunde wird die Festigkeit üblicherweise mit einem einfachen Prüfverfahren ermittelt, das sich gut für Vergleichsuntersuchungen eignet. In diesem Prüfverfahren wird ein Prüfstempel mit einem definierten Durchmesser in die Schaumkeramik eingedrückt und die Kraft-Weg-Kurve wird aufgezeichnet. Die zur Zerstörung der Struktur benötigte Kraft wird als Mass für die Festigkeit angenommen. Sie wird als Bruchlast in der Einheit N angegeben und als Mittelwert aus einem Los von zehn Proben ermittelt. Durch den Stempeleindruck ist die Prüfung definierter Probenbereiche möglich, ohne dass der für Schaumkeramik typische Festigkeitsgradient in den Randzonen in das Ergebnis eingeht.

Für die Bestimmung der Festigkeit der erfindungsgemässen Knochen-Ersatzmaterialien wird eine übliche Prüfmaschine (z.B. der Firma Instron) und ein Prüfstempel mit einem Durchmesser von 20mm verwendet. Jeder Prüfkörper soll quer zur Bruchlast mindestens 40 x 40 mm messen und mindestens 10 mm dick sein.

Als Bruchlast wird der erste Peak der Kraft-Weg-Kurve verwendet. Dieser ist bedingt durch das Einbrechen der obersten Stegebene. Alle weiteren Peaks, die der Zerstörung innerer Probenbereiche zuzuordnen sind, werden überlagert von Verdichtungsvorgängen und liefern keine verwertbaren Aussagen für die Festigkeit.

Die Höhe der gemessenen Bruchlast ist auf der Seite des Prüfkörpers abhängig vom Keramikmaterial, von der Porosität, von der Qualität der Schaumstruktur (Gleichmässigkeit der Stege, Risse etc.) und auf der Seite der Prüfmaschine von der Fläche des Prüfstempels und der Geschwindigkeit der Krafterhöhung.

Das erfindungsgemässe Verfahren und die durch das erfindungsgemässe Verfahren hergestellten Calziumphosphat-Schäume werden anhand der folgenden Figuren im Detail beschrieben. Dabei zeigen:
- **Figur 1**: ein idealisiertes Detail aus der Struktur eines retikulierten Kunststoffschaumes oder einer daraus hergestellten Schaumkeramik;
- **Figur 2**: einen schematischen Schnitt durch eine Strebe einer Schaumkeramik, die durch zweimaligen Durchgang durch das Schwartzwalder-Verfahren hergestellt wurde;
- **Figuren**: **3 und 4** zwei vergrösserte Schnitte durch ein Knochen-Ersatzmaterial, das durch zweimaligen Durchgang durch das Schwartzwalder-Verfahren hergestellt wurde.

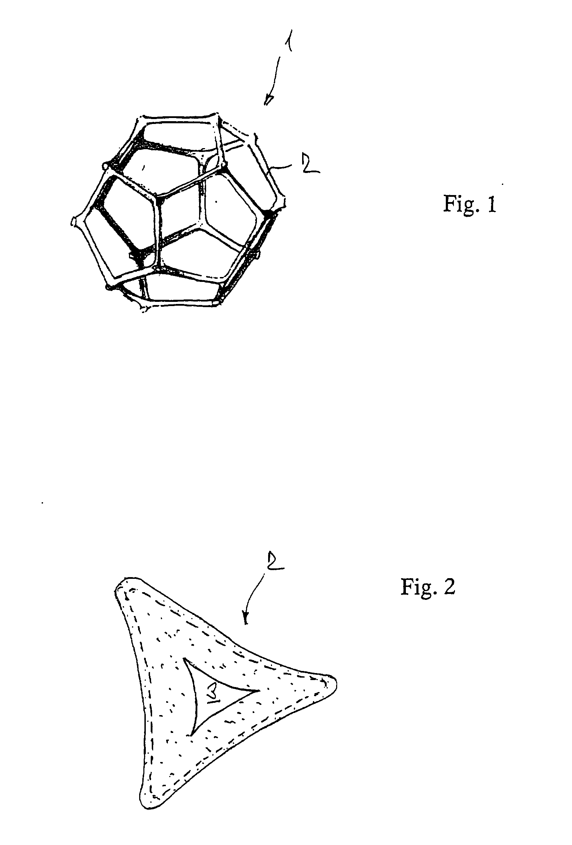

**Figur 1** zeigt eine Gerüstzelle 1 eines retikulierten Kunststoffschaumes oder einer daraus hergestellten Schaumkeramik. Die dargestellte Gerüstzelle ist stark idealisiert und basiert auf der Annahme dass alle Zellen des Schaumes genau gleich gross sind. Jede Zelle hat dann die Form eines Pentagondodekaeders und grenzt mit jeder ihrer Flächen an eine benachbarte Zelle an. Die Streben 2 bilden die Kanten des Pentagondodekaeders. Die Poren sind die Innenräume und sind über die Pentagondodekaeder-Flächen dreidimensional miteinander vernetzt.

**Figur 2** ist ein Querschnitt durch eine Strebe 2 einer durch zweimaligen Durchgang durch das Schwartzwalder-Verfahren hergestellten Schaumkeramik. Dieser Querschnitt ist aus geometrischen Gründen etwa dreieckig mit konkaven Seiten und schliesst einen Hohlraum 3 mit im wesentlichen derselben Form ein. Der Hohlraum nimmt die Position einer Strebe des ursprünglich eingesetzten und dann ausgebrannten Kunststoffschaumes ein. Die Strebe besteht aus einer inneren Keramikschicht, die von der ersten Beschlickerung stammt, und einer äusseren Schicht, die von der zweiten Beschlickerung stammt. Die beiden Schichten bestehen aus im wesentlichen demselben Calziumphosphat-Material, können sich aber in der Partikelgrösse unterscheiden.

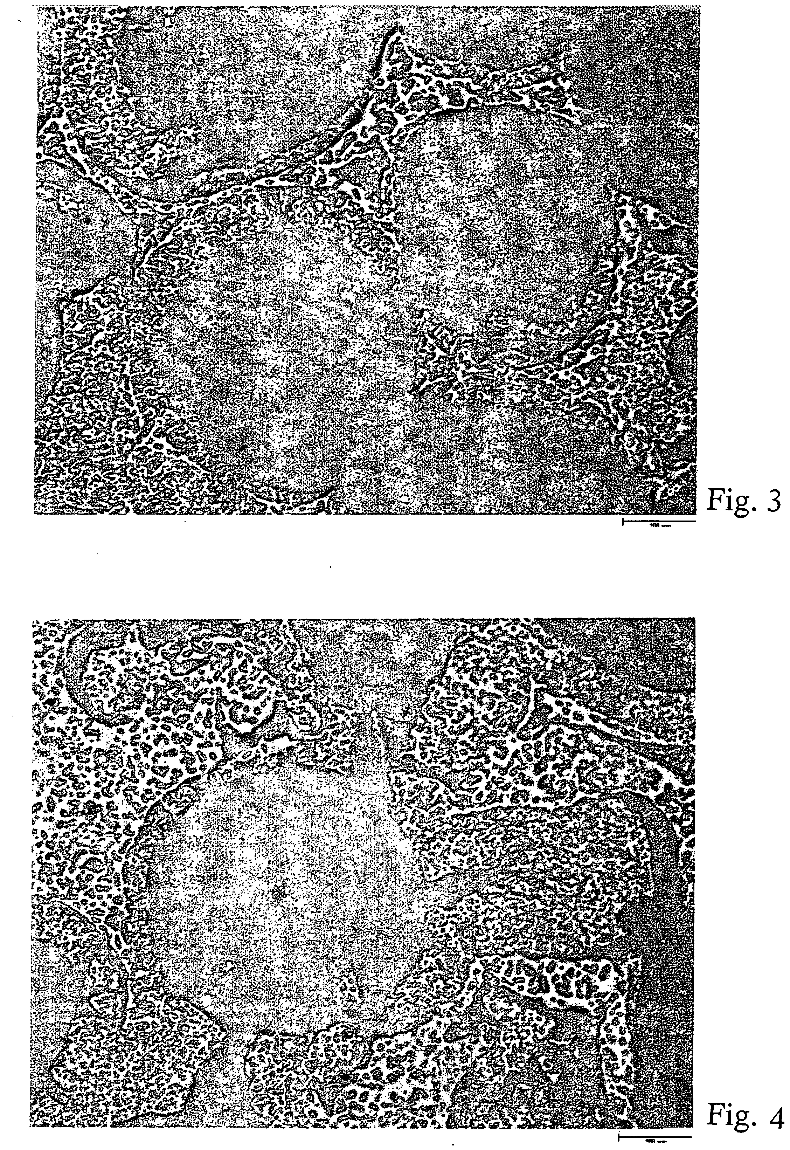

**Figuren 3 und 4** zeigen zwei Stellen eines mikroskopischen Schnittes durch eine Schaumkeramik aus Tri-Calziumphosphat, die durch zweimaligen Durchgang durch das Schwartzwalder-Verfahren hergestellt ist. Die zerklüftete Sinterstruktur ist in den beiden Figuren klar sichtbar und auch die Zweischichtigkeit der Streben, wobei die Sinterstruktur der inneren Schicht durch die zweimalige Sinterung sichtbar gröber ist als die Struktur der nur einmal gesinterten, äusseren Schicht. Die dargestellte Struktur stammt von einem Keramikschaum, der aus einer Mischung von Partikeln mit Partikelgrössen von 7µm und 3µm (Mischverhältnis 30:70) aus reinem α-Tri-Calziumphosphat (vorgesintert bei ca. 1300 bis 1350°C) hergestellt ist.

Zur Herstellung einer Schaumkeramik aus Tri-Calziumphosphat, die sich als abbaubares Knochen-Ersatzmaterial eignet, wird ein retikulierter Kunststoffschaum, beispielsweise aus Polyurethan mit einer offenen Porosität von 45 bis 90 ppi verwendet, was einer Zellweite von ca. 300 bis 1100µm entspricht. Das Tri-Calziumphosphat wird mehrheitlich oder vollständig in einem vorgesinterten Zustand (kristallin und phasenrein) und mit einer Partikelgrösse von ca. 1 bis 20µm eingesetzt. Das Keramikpulver wird zusammen mit einem in der Keramikbranche üblichen, für die Anwendung auf dem medizinischen Bereich unbedenklichen Dispergiermittel (z.B. auf der Basis einer Carbonsäurezubereitung), in alkalischem Wasser dispergiert. Danach wird ein ebenfalls in der Keramikbranche übliches und für die medizinische Anwendung unbedenkliches Bindemittel (z.B. ein teilverseifter Polyvinylalkohol) zur Dispersion zugegeben. Der so zubereitete Schlicker hat einen Feststoffgehalt von 40 bis 70% (vorzugsweise zwischen 60 und 70%), wovon das Bindemittel 1 bis 10% ausmacht. Der Schlicker hat einen pH-Wert von ca. 10 bis 12 und eine Viskosität von ca. 1,5 bis 2,5 Ncm.

Der Polyurethanschaum wird beschlickert durch mehrmaliges Aufsaugenlassen und Auspressen des Schlickers. Der beschlickerte Schaum wird bei einer Temperatur von weniger als 100° getrocknet, bis er ein konstant bleibendes Gewicht hat. Dann wird er langsam auf 1000 bis 1300°C (Sintertemperatur niedriger als Vorsintertemperatur) erhitzt und für 20 Stunden gesintert. Der resultierende Schaum hat eine Porosität von ca. 80 bis 90%.

Der Schlicker für die zweite Beschlickerung wird im wesentlichen gleich hergestellt wie der Schlicker für die erste Beschlickerung, weist aber einen kleineren Anteil an Feststoffen von weniger als 40% (z.B. zwischen 30 und 40%) auf. Der zweite Schlicker wird mehrere Male aktiv durch die vorfabrizierte Schaumkeramik gesaugt. Trocknung und Sinterung werden im wesentlichen gleich durchgeführt wie das erste Mal. Die resultierende Schaumkeramik hat eine Porosität von ca. 70 bis 80%.

### Beispiel 1

Für die Herstellung von phasenreinem α Tri-Calziumphosphat wurde amorphes Tri-Calziumphosphat bei 1300 bis 1350°C vorgesintert. Das vorgesinterte Keramikmaterial wurde gemahlen und in Siebfraktionen aufgeteilt. Zu ausgesiebten Partikeln mit einer Partikelgrösse d₅₀ von 7,3µm wurden 2 Gewichtsteile Dispergiermittel zugegeben und 50 Gewichtsteile amonialkalisches Wasser (pH 11). Die Aufschlämmung wurde während einer Stunde in einer Kugelmühle deagglomeriert. Dann wurden die Mahlkugeln durch Sieben abgetrennt und dem Schlicker unter ständigem Weiterrühren eine Lösung von 2 Gewichtsteilen Bindemittel in ca. 4 Gewichtsteilen Wasser und einige Tropfen Entschäumer zugefügt. Mit dem so hergestellten Schlicker wurden aus einem retikulierten Polyurethanschaum mit einer Porosität von 80 ppi geschnittene Stücke mit Abmessungen von 50x50x10mm behandelt. Überschüssiger Schlicker wurde durch Passieren zwischen zwei Pressrollen ausgepresst. Dann wurden die beschlickerten Stücke während 2 Stunden bei 80° C getrocknet und anschliessend langsam auf 1250°C erhitzt, während 20 Stunden auf dieser Temperatur gesintert und dann langsam ausgekühlt. Die Schaumkeramik-Stücke hatten ein Volumen, das gegenüber den Polyurethanschaum-Stücken um ca. 6% geschwunden war, ein Gewicht (Sintergewicht) von zwischen 5,6 und 6,6g und eine Porosität von 80 bis 88%.

Für die zweite Beschlickerung wurde nach demselben Verfahren, wie oben für den ersten Schlicker beschrieben, ein zweiter Schlicker hergestellt, wobei der Feststoffgehalt durch Zugabe von mehr Wasser auf 38% beschränkt wurde. Die Schaumkeramik-Stücke wurden in eine Nutsche gelegt und mit Schlicker begossen und dabei ständig abgesaugt. Dies wurde mehrmals wiederholt, wobei die Stücke jedesmal gedreht wurden. Dann wurden die Stücke wiederum getrocknet und gesintert. Die durch zweimalige Beschlickerung hergestellten Schaumkeramik-Stücke hatten ein Sintergewicht von zwischen 11,2 und 12,8 g und eine Porosität von 76 und 80%, wobei die Porenabmessungen immer noch deutlich über 100µm lagen.

Die nach der weiter oben beschriebenen Messmethode gemessene Kaltdruckfestigkeit (Bruchlast) betrug für die nach dem oben beschriebenen Vorgehen hergestellten Schaumkeramik-Stücke 660 N (Mittelwert von 10 Einzelmessungen).

### Beispiel 2

Ausgesiebte Partikel aus reinem α Tri-Calziumphosphat mit Partikelgrössen d₅₀ von einerseits 12µm und andererseits 2µm wurden im Gewichtsverhältnis von 30:70 gemischt. Aus dieser Mischung wurde wie im Beispiel 1 beschrieben ein Schlicker hergestellt und mit dem Schlicker ein retikulierter Polyurethanschaum beschlickert.

Dieser wurde wie im Beispiel 1 beschreiben getrocknet und gesintert. Der in diesem einmaligen Durchgang durch das Schwartzwalder-Verfahren erzeugte Keramikschaum wies eine Festigkeit bzw. Bruchlast von 610 N auf.

### Beispiel 3 (Vergleich)

Eine Mischung von vorgesintertem Tri-Calziumphosphat (reine α-Phase, d₅₀: 7µm, Gewichtsanteil 60%) und nicht vorgesintertem Tri-Calziumphosphat (amorph, d₅₀: 1µm, Gewichtsanteil 40%) wurde wie im Beispiel 2 beschrieben in einem einmaligen Durchgang durch das Schwartzwalder-Verfahren zu einer Schaumkeramik verarbeitet. Diese wies eine Festigkeit bzw. Bruchlast von 300 N auf.

## Patentansprüche

1. Knochen-Ersatzmaterial, das aus Calziumphosphat besteht, das nach einem Schwartzwalder-Verfahren ausgehend von einem retikulierten Kunststoffschaum mit einer Porosität im Bereiche von 18 bis 36 Poren pro cm (45 bis 90 ppi) hergestellt ist und das eine entsprechende Struktur mit einer offenen Porosität von mindestens 70 Vol.% aufweist, **dadurch gekennzeichnet, dass** das Material eine als Bruchlast mit einem Stempel von 20mm Durchmesser ermittelte Festigkeit von mindestens 550 N aufweist, dadurch, dass es vollständig kristallin und phasenrein ist und/oder Streben (2) mit einer mindestens zweischichtigen Struktur aufweist.

2. Knochen-Ersatzmaterial nach Anspruch 1, **dadurch gekennzeichnet, dass** es aus Calzium-Metaphosphat (Ca(PO₃)₂), Di-Calziumphosphat (Ca₂P₂O₇), Tri-Calziumphosphat (Ca₃(PO₄)₂), Hydroxyapatit (Ca₅(PO₄)₃OH) oder Tetra-Calziumphosphat (Ca₄(PO₄)₂O) besteht.

3. Knochen-Ersatzmaterial nach einem der Ansprüche 1 oder 2, **dadurch gekennzeichnet, dass** es aus α Tri-Calziumphosphat besteht.

4. Verfahren zur Herstellung eines offenporigen Knochen-Ersatzmaterials, wobei ein retikulierter Schaum aus einem Kunststoff mit einem ersten Schlicker aus einem Calziumphosphat-Pulver beschlickert, der beschlickerte Schaum getrocknet, der Kunststoff ausgebrannt und das verbleibende Calziumphosphat gesintert wird, wobei eine offenporige Schaumkeramik entsteht, **dadurch gekennzeichnet, dass** zur Erhöhung der Festigkeit der Schaumkeramik das Calziumphosphat-Pulver aus einem vollständig kristallinen und phasenreinen Calziumphosphat besteht oder dass das Pulver aus einem mindestens mehrheitlich aus kristallinem und phasenreinem Calziumphosphat besteht und die offenporige Schaumkeramik mit einem zweiten Schlicker aus einem zweiten Pulver desselben Calziumphosphats beschlickert, wiederum getrocknet und nochmals gesintert wird, wobei die Sintertemperatur in allen Fällen derart eingestellt wird, dass während der Sinterung keine Phasenumwandlung ausgelöst wird.

5. Verfahren nach Anspruch 4, **dadurch gekennzeichnet, dass** das Calziumphosphat Calzium-Metaphosphat (Ca(PO₃)₂), Di-Calziumphosphat (Ca₂P₂O₇), Tri-Calziumphosphat (Ca₃(PO₄)₂), Hydroxyapatit (Ca₅(PO₄)₃OH) oder Tetra-Calziumphosphat (Ca₄(PO₄)₂O) ist.

6. Verfahren nach Anspruch 4, **dadurch gekennzeichnet, dass** das Calziumphosphat α Tri-Calziumphosphat ist, das durch Sinterung von amorphem Tri-Calziumphosphat durch Sinterung bei 1300-1350° hergestellt wird, und dass die Sintertemperatur zwischen 1000 und 1300°C eingestellt wird.

7. Verfahren nach einem der Ansprüche 4 bis 6, **dadurch gekennzeichnet, dass** der zweite Schlicker einen kleineren Festkörperanteil aufweist als der erste Schlicker.

8. Verfahren nach Anspruch 7, **dadurch gekennzeichnet, dass** der erste Schlikker einen Festkörperanteil von 60 bis 70% und der zweite Schlicker einen Festkörperanteil von 30 bis 40% aufweist.

9. Verfahren nach einem der Ansprüche 4 bis 8, **dadurch gekennzeichnet, dass** für die Herstellung des ersten und des zweiten Schlickers ein Calziumphosphat-Pulver mit einer Partikelgrösse von 1 bis 20µm verwendet wird.

10. Verfahren nach einem der Ansprüche 4 bis 8, **dadurch gekennzeichnet, dass** das Calziumphosphat-Pulver einerseits Partikel einer Partikelgrösse von 1 - 5 µm und andererseits Partikel einer Partikelgrösse von 7 - 15 µm enthält.

11. Verfahren nach einem der Ansprüche 4 bis 10, **dadurch gekennzeichnet, dass** die zweite Beschlickerung mit Trocknung und Sinterung mindestens einmal wiederholt wird.

12. Knochen-Ersatzmaterial nach einem der Ansprüche 1 bis 3 für die Verwendung als Formstück zur Reparatur von Knochendefekten in belasteten oder teilbelasteten Bereichen.

13. Verwendung eines Knochen-Ersatzmaterials nach einem der Ansprüche 1 bis 6 zur Herstellung von Formstücken mittels mechanischer Bearbeitung.

## Claims

1. A bone replacement material consisting of calcium phosphate and manufactured according to a Schwartzwalder method starting from a reticulated plastic foam with a porosity in the region of 18 to 36 pores per cm (45 to 90 ppi), and having a corresponding structure with an open porosity of at least 70 % by volume, **characterised in that** the material has a strength of at least 550 N determined as a breaking load with a punch of 20 mm diameter by being completely crystalline and phase-pure and/or by comprising struts (2) of a two layered structure.

2. The bone replacement material according to claim 1, **characterised in that** it consists of calcium metaphosphate (Ca(PO₃)₂), di-calcium phosphate (Ca₂P₂O₇), tri-calcium phosphate (Ca₃(PO₄)₂), hydroxyapatite (Ca₅(PO₄)₃OH) or tetra calcium phosphate (Ca₄(PO₄)₂O).

3. The bone replacement material according to one of claims 1 or 2, **characterised in that** it consists of α tri-calcium phosphate.

4. A method for manufacturing an open-pored bone replacement material, wherein a reticulated foam of a plastic material is slurried with a first slurry of a calcium phosphate powder, the slurried foam is dried, the plastic is burnt out and the remaining calcium phosphate is sintered, whereby an open-pored foam ceramic is produced, **characterised in that** for increasing the strength of the foam ceramic, the calcium phosphate powder consists of a completely crystalline and phase-pure calcium phosphate or the powder consists of an at least majority crystalline and phase-pure calcium phosphate, and the open-pored foam ceramic is slurried with a second slurry of a second powder of the same calcium phosphate, is then dried and sintered once again, wherein the sinter temperature in all cases is set in a manner such that no phase change is initiated during sintering.

5. The method according to claim 4, **characterised in that** the calcium phosphate is calcium metaphosphate (Ca(PO₃)₂), di-calcium phosphate (Ca₂P₂O₇), tri-calcium phosphate (Ca₃(PO₄)₂), hydroxyapatite (Ca₅(PO₄)₃OH) and tetra-calcium phosphate (Ca₄(PO₄)₂O).

6. The method according to claim 4, **characterised in that** the calcium phosphate is α tri-calcium phosphate which is manufactured by pre-sintering amorphous tri-calcium phosphate at 1300-1350°, and that the sintering temperature is set between 1000 and 1300 °C.

7. The method according to one of claims 4 to 6, **characterised in that** the second slurry has a smaller solid body content than the first slurry.

8. The method according to claim 7, **characterised in that** the first slurry has a solid body content of 60 to 70 % and the second slurry has a solid body content of 30 to 40%.

9. The method according to one of claims 4 to 8, **characterised in that** a calcium phosphate powder with a particle size of 1 to 20 µm is used for manufacturing the first and the second slurry.

10. The method according to one of the claims 4 to 8, **characterised in that** the calcium phosphate powder on the one hand contains particles of a particle size of 1-5 µm and on the other hand particles of a particle size of 7-15 µm.

11. The method according to one of claims 4 to 10, **characterised in that** the second slurrying with drying and sintering is repeated at least once.

12. The bone replacement material according to one of claims 1 to 3 for the use as a shape piece for the repair of a bone defect in loaded or partly loaded regions.

13. Use of a bone replacement material according to one of the claims 1 to 6 for manufacturing shape pieces by way of mechanical processing.

## Revendications

1. Matériau de substitution osseux constitué de phosphate de calcium, qui est fabriqué selon le procédé de Schwartzwalder à partir d'une mousse plastique réticulée ayant une porosité comprise dans la gamme de 18 à 36 pores au cm (45 à 90 ppi), et qui présente une structure correspondante avec une porosité ouverte d'au moins 70 % en volume, **caractérisé en ce que** le matériau présente une solidité d'au moins 550 N, exprimée en tant que charge à la rupture déterminée avec un poinçon d'un diamètre de 20 mm, **en ce qu'**il est entièrement cristallin et exempt d'impuretés de phase et/ou **en ce qu'**il présente des montants (2) ayant une structure à au moins deux couches.

2. Matériau de substitution osseux selon la revendication 1, **caractérisé en ce qu'**il est constitué de métaphosphate de calcium (Ca(PO₃)₂), de phosphate dicalcique (Ca₂P₂O₇), de phosphate tricalcique (Ca₃(PO₄)₂), d'hydroxyapatite (Cas(PO₄)₃OH) ou de phosphate tétracalcique (Ca₄(PO₄)₂O).

3. Matériau de substitution osseux selon l'une quelconque des revendications 1 ou 2, **caractérisé en ce qu'**il est constitué de phosphate tricalcique α.

4. Procédé de fabrication d'un matériau de substitution osseux à pores ouverts, dans lequel une mousse réticulée à base d'un matériau plastique est imprégnée avec une première suspension à base d'une poudre de phosphate de calcium, la mousse imprégnée est séchée, le matériau plastique est calciné et le phosphate de calcium subsistant est fritté, une mousse céramique à pores ouverts étant ainsi produite, **caractérisé en ce que**, pour augmenter la solidité de la mousse céramique, la poudre de phosphate de calcium se compose d'un phosphate de calcium entièrement cristallin et exempt d'impuretés de phase ou **en ce que** la poudre se compose d'un phosphate de calcium au moins majoritairement cristallin et exempt d'impuretés de phase et la mousse céramique à pores ouverts est imprégnée avec une deuxième suspension à base d'une deuxième poudre du même phosphate de calcium, à nouveau séchée et une nouvelle fois frittée, la température de frittage étant réglée dans tous les cas de telle sorte qu'aucun changement de phase n'est déclenché pendant le frittage.

5. Procédé selon la revendication 4, **caractérisé en ce que** le phosphate de calcium est un métaphosphate de calcium (Ca(PO₃)₂), un phosphate dicalcique (Ca₂P₂O₇), un phosphate tricalcique (Ca₃(PO₄)₂), une hydroxyapatite (Ca₅(PO₄)₃OH) ou un phosphate de tétracalcique (Ca₄(PO₄)₂O).

6. Procédé selon la revendication 4, **caractérisé en ce que** le phosphate de calcium est un phosphate tricalcique α qui est fabriqué par frittage de phosphate tricalcique amorphe par frittage à 1300-1350 C et **en ce que** la température de frittage est réglée entre 1000 et 1300 °C.

7. Procédé selon l'une quelconque des revendications 4 à 6, **caractérisé en ce que** la deuxième suspension présente une teneur en matières solides inférieure à celle de la première suspension.

8. Procédé selon la revendication 7, **caractérisé en ce que** la première suspension présente une teneur en matières solides de 60 à 70 % et la deuxième suspension une teneur en matières solides de 30 à 40%.

9. Procédé selon l'une quelconque des revendications 4 à 8, **caractérisé en ce qu'**une poudre de phosphate de calcium avec une taille de particules de 1 à 20 µm est utilisée pour la préparation des première et deuxième suspensions.

10. Procédé selon l'une quelconque des revendications 4 à 8, **caractérisé en ce que** la poudre de phosphate de calcium contient, d'une part, des particules d'une taille allant de 1 à 5 µm et, d'autre part, des particules d'une taille allant de 7 à 15 µm.

11. Procédé selon l'une quelconque des revendications 4 à 10, **caractérisé en ce que** la deuxième imprégnation suivie du séchage et du frittage est recommencée au moins une fois.

12. Matériau de substitution osseux selon l'une quelconque des revendications 1 à 3 pour l'utilisation en tant que pièce façonnée aux fins de la réparation de défauts osseux dans des zones soumises à des charges ou à des charges partielles.

13. Utilisation d'un matériau de substitution osseux selon l'une quelconque des revendications 1 à 6 aux fins de la fabrication de pièces façonnées au moyen d'un traitement mécanique.
